# EUROPEAN PATENT SPECIFICATION

(11) **EP 2 082 369 B1**
(45) Date of publication and mention of the grant of the patent: **08.03.2017**
(21) Application number: 07843168.1
(22) Date of filing: 25.09.2007
(51) Int. Cl.: G06T 5/00, A61B 8/12, A61B 5/00, G10K 11/35, G01S 15/89, A61B 8/08, G01S 7/531

(54) **SYSTEMS AND METHODS FOR RESTORING A MEDICAL IMAGE AFFECTED BY NONUNIFORM ROTATIONAL DISTORTION**
SYSTEME UND VERFAHREN ZUR WIEDERHERSTELLUNG EINES DURCH UNGLEICHFÖRMIGE ROTATIONSVERZERRUNG BEEINTRÄCHTIGTEN MEDIZINISCHEN BILDES
SYSTÈMES ET PROCÉDÉS POUR RESTAURER UNE IMAGE MÉDICALE AFFECTÉE PAR UNE DISTORSION DE ROTATION NON UNIFORME

(30) Priority: 26.09.2006 US 535441
(43) Date of publication of application: 29.07.2009
(73) Proprietor: Boston Scientific Limited, Hamilton HM11 (BM)
(72) Inventor: LAM, Duc, San Jose, California 95148 (US); SATHYANARAYANA, Shashidhar, Pleasanton, California 94566 (US); TEO, Tat-jin, Maple Grove, California 55311-1566 (US)
(74) Representative: Vossius & Partner Patentanwälte Rechtsanwälte mbB
(86) International application number: PCT/US2007/079448
(87) International publication number: WO 2008/039793

(56) References cited:
- US-A- 4 374 525
- US-A- 4 850 363
- US-A- 5 485 845
- US-A- 6 019 726
- US-A1- 2003 147 551
- US-B1- 6 592 526

## Description

### FIELD OF THE INVENTION

The field of the invention relates to medical imaging systems, and more particularly to systems and methods for restoring a medical image affected by nonuniform rotational distortion.

### BACKGROUND OF THE INVENTION

For purposes of diagnosis and treatment planning, imaging techniques such as ultrasound imaging are commonly used in medical procedures to obtain images of the inside of a patient's body. In intravascular ultrasound (IVUS) imaging, images revealing the internal anatomy of blood vessels are obtained by inserting a catheter with an ultrasound transducer mounted on or near its tip into the blood vessel. The ultrasound transducer is positioned in a region of the blood vessel to be imaged, where it emits pulses of ultrasound energy. The pulses reflect off of the blood vessel wall and surrounding tissue and return back to the transducer. The reflected ultrasound energy (echo) impinging on the transducer produces an electrical signal, which is used to form an image of the blood vessel.

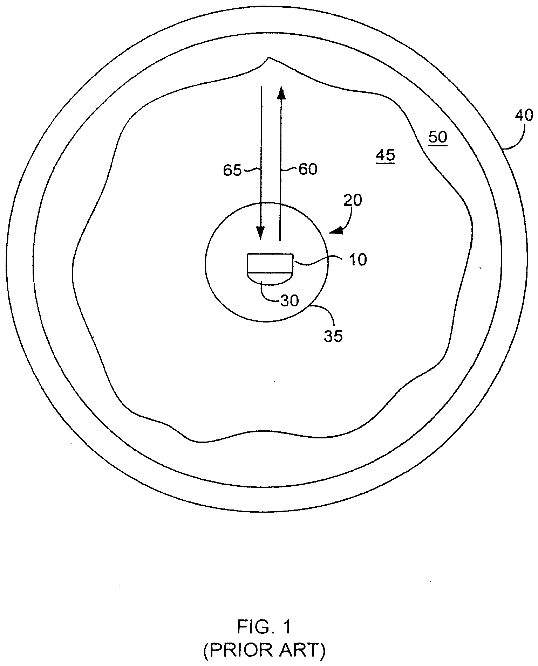

To obtain a cross-sectional image or "slice" of the blood vessel, the transducer must interrogate the vessel in all directions. This can be accomplished by mechanically rotating the transducer during imaging. Fig. 1 is a representation of an axial view of a rotating transducer 10 mounted on the tip of a prior art catheter 20. The transducer 10 is coupled to a drive motor (not shown) via a drive cable 30 and rotates within a sheath 35 of the catheter 20. The blood vessel 40 being imaged typically includes a blood region 45 and wall structures (blood-wall interface) 50 and the surrounding tissue.

A cross-sectional image of the blood vessel is obtained by having the transducer 10 emit a plurality of ultrasound pulses, e.g., 256, at different angles as it is rotated over one revolution. Fig. 1 illustrates one exemplary ultrasound pulse 60 being emitted from the transducer 10. The echo pulse 65 for each emitted pulse 60 received by the transducer is used to compose one radial line or "image vector" in the image of the blood vessel. Ideally, the transducer 10 is rotated at a uniform angular velocity so that the image vectors are taken at evenly spaced angles within the blood vessel 40. An image processor (not shown) assembles the image vectors acquired during one revolution of the transducer 10 into a cross-sectional image of the blood vessel 40. The image processor assembles the image vectors based on the assumption that the image vectors were taken at evenly spaced angles within the blood vessel 40, which occurs when the transducer 10 is rotated at uniform angular velocity.

Unfortunately, it is difficult to achieve and maintain a uniform angular velocity for the transducer 10. This is because the transducer 10 is mechanically coupled to a drive motor (not shown), which may be located one to two meters from the transducer, via the drive cable 30. The drive cable 30 must follow all the bends along the path of the blood vessel to reach the region of the blood vessel 40 being imaged. As a result, the drive cable 30 typically binds and/or whips around as it is rotated in the blood vessel 40. This causes the transducer 10 to rotate at a nonuniform angular velocity even though the motor rotates at a uniform angular velocity. This is a problem because the angles assumed by the image processor in assembling the image vectors into the cross-sectional image of the blood vessel 40 are different from the actual angles at which the image vectors were taken. This causes the cross-sectional image of the blood vessel to be distorted in the azimuthal direction. The resulting distortion is referred as Nonuniform Rotational Distortion (NURD).

US 2003/0147551 A1 discloses a medical imaging system comprising an imaging catheter having proximal and distal sections, an imaging device coupled to the distal section of the imaging catheter, said imaging device configured to rotate at a uniform angular velocity and comprising a single transducer configured to emit energy pulses. In order to reduce NURD, a processor connected to the medical imaging system receives input images comprising a plurality of image vectors, computes a spectral measure of the texture around each pixel by performing a Fourier transform within a weighed window centered at the pixel, computes the mean frequency of the Fourier transform for each pixel, and computes for each image vector the average of the mean frequency for the pixels in the image vector. The estimate of the actual angle is computed for each image vector using the running value of a normalized integral at the image vector, and the plurality of vectors are remapped according to the respective estimated angles.

US 5,485,845 discloses a similar apparatus utilizing several beacons to minimize NURD.

US 4,374,525 discloses a medical imaging system comprising an imaging device comprising a first and second imaging transducer, but shows no connnection to NURD.

Therefore, there is need for an image processing technique that reduces NURD in IVUS images acquired using a rotating transducer.

### SUMMARY OF THE INVENTION

The field of the invention relates to medical imaging systems, and more particularly to systems and methods for restoring a medical image affected by nonuniform rotational distortion.

One embodiment of the invention is an imaging system according to claim 1.

Another embodiment of the invention is a process for reducing non-uniform rotational distortion in a medical image according to claim 7.

Other systems, methods, features and advantages of the invention will be or will become apparent to one with skill in the art upon examination of the following figures and detailed description. It is intended that all such additional systems, methods, features and advantages be included within this description, be within the scope of the invention, and be protected by the accompanying claims.

### BRIEF DESCRIPTION OF THE DRAWINGS

In order to better appreciate how the above-recited and other advantages and objects of the inventions are obtained, a more particular description of the embodiments briefly described above will be rendered by reference to specific embodiments thereof, which are illustrated in the accompanying drawings. It should be noted that the components in the figures are not necessarily to scale, emphasis instead being placed upon illustrating the principles of the invention. Moreover, in the figures, like reference numerals designate corresponding parts throughout the different views. However, like parts do not always have like reference numerals. Moreover, all illustrations are intended to convey concepts, where relative sizes, shapes and other detailed attributes may be illustrated schematically rather than literally or precisely.
Fig. 1 is a representation of a rotating transducer of a prior art catheter inside a blood vessel;
Fig. 2 is a representation of a rotating imaging device in accordance with an embodiment of the present invention; and
Fig. 3 is a diagram of a process in accordance with an embodiment of the present invention.

### DETAILED DESCRIPTION OF THE PREFERRED EMBODIMENTS

Described below is a new image processing method that reduces NURD in medical images, such as IVUS images, acquired using a rotating imaging device. In the case of a rotating imaging device, such as a transducer or light emitting device (e.g., using optical coherence tomography), at the tip of a catheter, one approach to reduce NURD is to estimate the instantaneous angular velocity of the imaging device as it rotates to determine the amount of distortion. This approach may be achieved by using a plurality of imaging devices, such as ultrasound imaging transducers. As mentioned above, other imaging devices may be used, instead of, or in addition to imaging transducers, such as apparatuses for obtaining images through optical coherence tomography (OCT). Image acquisition using OCT is described in Huang et al., "Optical Coherence Tomography," Science, 254, Nov. 22, 1991, pp 1178-1181. A type of OCT imaging device, called an optical coherence domain reflectometer (OCDR) is disclosed in Swanson U.S. Pat. No. 5,321,501. The OCDR is capable of electronically performing two- and three-dimensional image scans over an extended longitudinal or depth range with sharp focus and high resolution and sensitivity over the range.

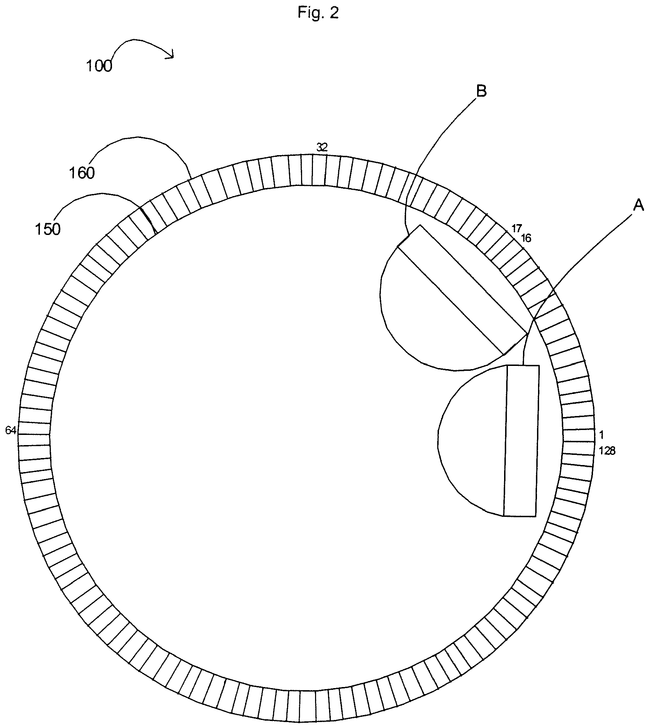

Turning to Fig. 2, an axial view of an imaging device 100 mounted on the tip of a catheter 150 is shown. The imaging device 100 includes two imaging devices A and B, illustrated as transducers in this example embodiment, positioned such that they each emit energy pulses at generally right angles to the axis of the catheter 150. Further, the imaging transducers A and B are also positioned at a 45° angle with respect to each other. In accordance with another embodiment, A and B may be positioned at any angle with respect to each other.

As mentioned above, a cross-sectional image of the blood vessel is obtained by having the imaging device 100 emit a plurality of ultrasound pulses, e.g., 256 or 128, at different angles as it is rotated over one revolution. The echo signals received from the emitted pulses are typically classified by records, or vectors, corresponding to a particular angular position in a revolution.

In Fig. 2, the imaging device 100 is overlaid onto a chart that maps 128 vectors in one revolution. In this embodiment, each transducer, A and B, generates at least 128 vectors in one revolution. During operation of the imaging device 100, in the absence of NURD, i.e., when the imaging device 100 rotates at a uniform angular velocity, as the imaging device 100 rotates, vector 1, generated by transducer A would be essentially identical to vector 17, generated by transducer B. In other words, a search of the vectors generated by transducer B would reveal that vector 17 correlates most strongly with vector 1 of transducer A.

When the imaging device 100 does not rotate at a uniform angular velocity, NURD may occur and the instantaneous angular velocity of the imaging device 100 may differ from the expected uniform angular velocity. In such a case, a search of the vectors of generated by transducer B would reveal that vector 17 no longer most strongly correlates with vector 1 of transducer A. One of ordinary skill in the art will appreciate that the discrepancy between the vector generated by transducer B that most strongly correlates with vector 1 of transducer A and the vector that would most strongly correlate with vector 1 of transducer A in the absence of NURD, e.g., vector 17, may be used to estimate the instantaneous angular velocity of the imaging device 100.

The estimated instantaneous angular velocity of the imaging device 100 may be used to remap the generated vectors to account for the discrepancy between the instantaneous angular velocity and the expected uniform angular velocity, which would, in effect, reduce the NURD in the resulting cross-sectional image. For example, if in the absence of NURD, vector 17 generated by transducer B most strongly correlates with vector 1 generated by transducer A, and if during the occurrence of NURD, vector 19 generated by transducer B most strongly correlates with vector 1 generated by transducer A, then the generated vectors that create the cross-sectional image may be remapped to account for the discrepancy between vector 17 and vector 19 generated by transducer B.

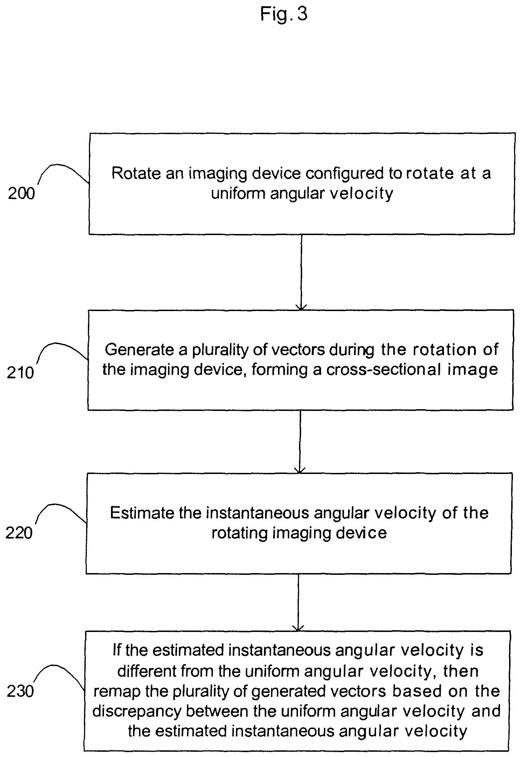

Turning to Fig. 3, a method for reducing NURD in a cross-sectional image of a lumen generated by an imaging device configured to rotate at a uniform angular velocity is shown. As the imaging device rotates (action block 200), a plurality of vectors are generated, forming the cross-sectional image (action block 210). In accordance with the method, a processor (not shown) may estimate an instantaneous angular velocity of the imaging device (action block 220). If the estimated instantaneous angular velocity differs from the uniform angular velocity, then the plurality of generated vectors may be remapped based on the discrepancy between the estimated instantaneous angular velocity and the uniform angular velocity (action block 230).

In the foregoing specification, the invention has been described with reference to specific embodiments thereof. It will, however, be evident that various modifications and changes may be made thereto without departing from the scope of the invention.

## Claims

1. A medical imaging system comprising:
an imaging catheter (150) having proximal and distal sections;
an imaging device (100) coupled to the distal section of the imaging catheter, said imaging device configured to rotate at a uniform angular velocity, the imaging device comprising a first imaging transducer (A) and a second imaging transducer (B) configured to emit energy pulses; and
a computer-usable medium, electrically coupled to the imaging device, having a sequence of instructions which, when executed by a processor, causes said processor to execute a process including generating a plurality of vectors (1-128), for each of the first and second imaging transducers, as the imaging device rotates to form an image, estimating an instantaneous angular velocity of the imaging device as the imaging device rotates using a discrepancy between a vector of the plurality of vectors generated by the first imaging transducer and a corresponding vector of the plurality of vectors generated by the second imaging transducer which would most strongly correlate with the vector generated by the first imaging transducer in an absence of non-uniform angular velocity, and remapping the plurality of vectors whose estimated instantaneous angular velocity differs from the uniform angular velocity.

2. The medical imaging system of claim 1, wherein the first and second imaging transducers are positioned such that the first imaging transducer emits energy pulses at a 45 degree angle from the energy pulses emitted by the second imaging transducers.

3. The medical imaging system of claim 1, wherein the imaging transducers are ultrasound transducers or, wherein each of the first and second imaging transducers are configured to generate 256 vectors in one rotation.

4. The medical imaging system of claim 1, wherein the medical image is a cross-sectional image of a lumen or, wherein the imaging system is configured to obtain a cross-sectional image of a lumen.

5. The medical imaging system of claim 1, wherein each vector generated by the first imaging transducer has a value that is substantially similar to a vector generated by the second imaging transducer.

6. The medical imaging system of claim 5, wherein the computer-usable medium has a sequence of instructions which , when executed by the processor, cause the processor to execute a process including:
determining which vector of the second imaging transducer has a value that is substantially similar to a particular vector of the first imaging transducer, and
calculating the discrepancy between the determined vector and an expected vector, wherein the expected vector is a vector of the second imaging transducer that is expected to have a value that is substantially similar to the particular vector of the first imaging transducer.

7. A method for reducing non-uniform rotational distortion in a medical image, the method comprising:
providing a plurality of vectors, from each of a first imaging transducer and a second imaging transducer of an imaging device (210) configured to rotate at a uniform angular velocity (200), that form the medical image;
estimating an instantaneous angular velocity of the imaging device using a discrepancy between one vector of the plurality of vectors from the first imaging transducer and a corresponding vector of the plurality of vectors from the second imaging transducer (220) which would most strongly correlate with the vector generated by the first imaging transducer in an absence of non-uniform angular velocity; and remapping the plurality of vectors if the instantaneous angular velocity differs from the uniform angular velocity (230).

8. The method of claim 7, wherein each of the first and second imaging transducers are configured to generate 256 vectors in one rotation or wherein the first and second imaging transducers are positioned at a 45 degree angle with respect to each other.

9. The method of claim 7, wherein each of the first and second imaging transducers are configured to generate a plurality of vectors.

10. The method of claim 9, wherein each vector generated by the first imaging transducer has a value that is substantially similar to a vector generated by the second imaging transducer.

11. The method of claim 10, wherein the step of estimating the instantaneous angular velocity comprises:
determining which vector of the second imaging transducer has a value that is substantially similar to a particular vector of the first imaging transducer, and
calculating the discrepancy between the determined vector and an expected vector, wherein the expected vector is a vector of the second imaging transducer that is expected to have a value that is substantially similar to the particular vector of the first imaging transducer.

## Patentansprüche

1. Medizinisches Bildgebungssystem, das aufweist:
einen Bildgebungskatheter (150), der proximale und distale Abschnitte aufweist;
eine Bildgebungsvorrichtung (100), die mit dem distalen Abschnitt des Bildgebungskatheters gekoppelt ist, wobei die Bildgebungsvorrichtung konfiguriert ist, sich mit einer gleichförmigen Winkelgeschwindigkeit zu drehen, wobei die Bildgebungsvorrichtung einen ersten Bildgebungswandler (A) und einen zweiten Bildgebungswandler (B) aufweist, die konfiguriert sind, Energieimpulse zu emittieren; und
ein computerverwendbares Medium, das mit der Bildgebungsvorrichtung elektrisch gekoppelt ist, das eine Abfolge von Befehlen aufweist, die, wenn sie durch einen Prozessor ausgeführt werden, den Prozessor veranlassen, einen Prozess auszuführen, der aufweist: Erzeugen von mehreren Vektoren (1-128) für jeweils den ersten und zweiten Bildgebungswandler, wenn sich die Bildgebungsvorrichtung dreht, um ein Bild aufzubauen, Schätzen einer augenblicklichen Winkelgeschwindigkeit der Bildgebungsvorrichtung, wenn sich die Bildgebungsvorrichtung dreht, mittels einer Diskrepanz zwischen einem Vektor der mehreren Vektoren, die durch den ersten Bildgebungswandler erzeugt werden, und einem entsprechenden Vektor der mehreren Vektoren, die durch den zweiten Bildgebungswandler erzeugt werden, der am stärksten mit dem Vektor korrelieren würde, der durch den ersten Bildgebungswandler beim Fehlen einer ungleichförmigen Winkelgeschwindigkeit erzeugt würde, und Neuzuordnen der mehreren Vektoren, deren geschätzte augenblickliche Winkelgeschwindigkeit sich von der gleichförmigen Winkelgeschwindigkeit unterscheidet.

2. Medizinisches Bildgebungssystem nach Anspruch 1, wobei der erste und zweite Bildgebungswandler so angeordnet sind, dass der erste Bildgebungswandler Energieimpulse unter einem Winkel von 45 Grad zu den Energieimpulsen emittiert, die durch den zweiten Bildgebungswandler emittiert werden.

3. Medizinisches Bildgebungssystem nach Anspruch 1, wobei die Bildgebungswandler Ultraschallwandler sind oder wobei jeweils der erste und zweite Bildgebungswandler konfiguriert sind, 256 Vektoren in einer Umdrehung zu erzeugen.

4. Medizinisches Bildgebungssystem nach Anspruch 1, wobei das medizinische Bild ein Querschnittbild eines Lumens ist oder wobei das Bildgebungssystem konfiguriert ist, ein Querschnittbild eines Lumens zu erhalten.

5. Medizinisches Bildgebungssystem nach Anspruch 1, wobei jeder durch den ersten Bildgebungswandler erzeugte Vektor einen Wert aufweist, der im Wesentlichen ähnlich zu einem durch den zweiten Bildgebungswandler erzeugten Vektor ist.

6. Medizinisches Bildgebungssystem nach Anspruch 5, wobei das computerverwendbare Medium eine Abfolge von Befehlen aufweist, die, wenn sie durch den Prozessor ausgeführt werden, den Prozessor veranlassen, einen Prozess auszuführen, der aufweist:
Feststellen, welcher Vektor des zweiten Bildgebungswandlers einen Wert aufweist, der zu einem bestimmten Vektor des ersten Bildgebungswandlers im Wesentlichen ähnlich ist, und
Berechnen der Diskrepanz zwischen dem festgestellten Vektor und einem erwarteten Vektor, wobei der erwartete Vektor ein Vektor des zweiten Bildgebungswandlers ist, von dem erwartet wird, dass er einen Wert aufweist, der zu dem bestimmten Vektor des ersten Bildgebungswandlers im Wesentlichen ähnlich ist.

7. Verfahren zum Reduzieren einer ungleichförmigen Rotationsverzerrung in einem medizinischen Bild, wobei das Verfahren aufweist:
Bereitstellen von mehreren Vektoren, die das medizinische Bild aufbauen, aus jeweils einem ersten Bildgebungswandler und einem zweiten Bildgebungswandler einer Bildgebungsvorrichtung (210), die konfiguriert ist, sich mit einer gleichförmigen Winkelgeschwindigkeit (200) zu drehen;
Schätzen einer augenblicklichen Winkelgeschwindigkeit der Bildgebungsvorrichtung mittels einer Diskrepanz zwischen einem Vektor der mehreren Vektoren aus dem ersten Bildgebungswandler und einem entsprechenden Vektor der mehreren Vektoren aus dem zweiten Bildgebungswandler (220), der am stärksten mit dem Vektor korrelieren würde, der durch den ersten Bildgebungswandler beim Fehlen einer ungleichförmigen Winkelgeschwindigkeit erzeugt würde; und
Neuzuordnen der mehreren Vektoren, wenn sich die augenblickliche Winkelgeschwindigkeit von der gleichförmigen Winkelgeschwindigkeit (230) unterscheidet.

8. Verfahren nach Anspruch 7, wobei jeweils der erste und zweite Bildgebungswandler konfiguriert sind, 256 Vektoren in einer Umdrehung zu erzeugen oder wobei der erste und zweite Bildgebungswandler unter einem Winkel von 45 Grad in Bezug zueinander angeordnet sind.

9. Verfahren nach Anspruch 7, wobei jeweils der erste und zweite Bildgebungswandler konfiguriert sind, mehrere Vektoren zu erzeugen.

10. Verfahren nach Anspruch 9, wobei jeder durch den ersten Bildgebungswandler erzeugte Vektor einen Wert aufweist, der im Wesentlichen ähnlich zu einem durch den zweiten Bildgebungswandler erzeugten Vektor ist.

11. Verfahren nach Anspruch 10, wobei der Schritt des Schätzens der augenblicklichen Winkelgeschwindigkeit aufweist:
Feststellen, welcher Vektor des zweiten Bildgebungswandlers einen Wert aufweist, der im Wesentlichen ähnlich zu einem bestimmten Vektor des ersten Bildgebungswandlers ist, und
Berechnen der Diskrepanz zwischen dem festgestellten Vektor und einem erwarteten Vektor, wobei der erwartete Vektor ein Vektor des zweiten Bildgebungswandlers ist, von dem erwartet wird, dass er einen Wert aufweist, der im Wesentlichen ähnlich zu dem bestimmten Vektor des ersten Bildgebungswandlers ist.

## Revendications

1. Système d'imagerie médicale, comprenant :
un cathéter d'imagerie (150) ayant une section proximale et une section distale ;
un dispositif d'imagerie (100) relié à la section distale du cathéter d'imagerie, ledit dispositif d'imagerie étant prévu pour tourner à une vitesse angulaire uniforme, ledit dispositif d'imagerie comprenant un premier transducteur d'imagerie (A) et un deuxième transducteur d'imagerie (B) prévus pour émettre des impulsions d'énergie ; et
un support lisible par ordinateur, relié électriquement au dispositif d'imagerie, comportant une séquence d'instructions qui, exécutée par un processeur, entraîne ledit processeur à exécuter un processus incluant : la génération d'une pluralité de vecteurs (1-128), pour le premier et pour le deuxième transducteurs d'imagerie quand le dispositif d'imagerie est entraîné en rotation pour former une image, l'estimation d'une vitesse angulaire momentanée du dispositif d'imagerie quand le dispositif d'imagerie est entraîné en rotation au moyen d'un écart entre un vecteur de la pluralité de vecteurs générés par le premier transducteur d'imagerie et un vecteur correspondant de la pluralité de vecteurs générés par le deuxième transducteur d'imagerie, lequel correspondrait le plus au vecteur généré par le premier transducteur d'imagerie en l'absence d'une vitesse angulaire non uniforme, et la nouvelle cartographie de la pluralité de vecteurs dont la vitesse angulaire momentanée estimée diffère de la vitesse angulaire uniforme.

2. Système d'imagerie médicale selon la revendication 1, où le premier et le deuxième transducteurs d'imagerie sont positionnés de telle manière que le premier transducteur d'imagerie émet des impulsions d'énergie suivant un angle de 45° par rapport aux impulsions d'énergie émises par le deuxième transducteur d'imagerie.

3. Système d'imagerie médicale selon la revendication 1, où les transducteurs d'imagerie sont des transducteurs ultrasonores, ou bien où le premier et le deuxième transducteurs d'imagerie sont prévus pour générer 256 vecteurs en une rotation.

4. Système d'imagerie médicale selon la revendication 1, où l'image médicale est une image en section transversale d'une lumière, ou bien où le système d'imagerie est prévu pour obtenir une image en section transversale d'une lumière.

5. Système d'imagerie médicale selon la revendication 1, où chaque vecteur généré par le premier transducteur d'imagerie a une valeur sensiblement similaire à celle d'un vecteur généré par le deuxième transducteur d'imagerie.

6. Système d'imagerie médicale selon la revendication 5, où le support lisible par ordinateur comporte une séquence d'instructions qui, exécutée par un processeur, entraîne ledit processeur à exécuter un processus incluant :
la détermination du vecteur du deuxième transducteur d'imagerie ayant une valeur sensiblement similaire à celle d'un vecteur particulier du premier transducteur d'imagerie, et
le calcul de l'écart entre le vecteur déterminée et un vecteur estimé, ledit vecteur estimé étant un vecteur du deuxième transducteur d'imagerie dont la valeur estimée est sensiblement similaire à la valeur du vecteur particulier du premier transducteur d'imagerie.

7. Procédé de réduction d'une distorsion de rotation non uniforme dans une image médicale, ledit procédé comprenant :
la génération d'une pluralité de vecteurs, d'un premier transducteur d'imagerie et d'un deuxième transducteur d'imagerie d'un dispositif d'imagerie (210) prévu pour tourner à une vitesse angulaire uniforme (200), qui forment l'image médicale ;
l'estimation d'une vitesse angulaire momentanée du dispositif d'imagerie au moyen d'un écart entre un vecteur de la pluralité de vecteurs du premier transducteur d'imagerie et un vecteur correspondant de la pluralité de vecteurs du deuxième transducteur d'imagerie (220), lequel correspondrait le plus au vecteur généré par le premier transducteur d'imagerie en l'absence d'une vitesse angulaire non uniforme ; et
la nouvelle cartographie de la pluralité de vecteurs si la vitesse angulaire momentanée diffère de la vitesse angulaire uniforme (230).

8. Procédé selon la revendication 7, où le premier et le deuxième transducteurs d'imagerie sont prévus pour générer 256 vecteurs en une rotation, ou bien où le premier et le deuxième transducteurs d'imagerie sont positionnés suivant un angle de 45° l'un par rapport à l'autre.

9. Procédé selon la revendication 7, où le premier et le deuxième transducteurs d'imagerie sont prévus pour générer une pluralité de vecteurs.

10. Procédé selon la revendication 9, où chaque vecteur généré par le premier transducteur d'imagerie a une valeur sensiblement similaire à celle d'un vecteur généré par le deuxième transducteur d'imagerie.

11. Procédé selon la revendication 10, où l'étape d'estimation de la vitesse angulaire momentanée comprend :
la détermination du vecteur du deuxième transducteur d'imagerie ayant une valeur sensiblement similaire à celle d'un vecteur particulier du premier transducteur d'imagerie, et
le calcul de l'écart entre le vecteur déterminée et un vecteur estimé, ledit vecteur estimé étant un vecteur du deuxième transducteur d'imagerie dont la valeur estimée est sensiblement similaire à la valeur du vecteur particulier du premier transducteur d'imagerie.
